# EUROPEAN PATENT SPECIFICATION

(11) **EP 2 510 981 B1**
(45) Date of publication and mention of the grant of the patent: **17.01.2018**
(21) Application number: 12158937.8
(22) Date of filing: 07.06.2002
(51) Int. Cl.: A61Q 11/00, A61K 8/73, A61K 8/44, A61K 8/60, A61K 8/43, A61K 8/41, A61K 8/49

(54) **Composition for oral cavity**
Zusammensetzungen für die Mundhöhle
Préparations pour la cavité buccale

(43) Date of publication of application: 17.10.2012
(62) Divisional of application: 02733375.6
(73) Proprietor: Sunstar Inc., Takatsuki-shi Osaka 569-1195 (JP)
(72) Inventor: NAKAO, Akira, Takatsuki-shi, Osaka 569-1044 (JP); Saito, Toru, Takatsuki-shi, Osaka 569-1044 (JP)
(74) Representative: Stoner, Gerard Patrick

(56) References cited:
- EP-A- 0 744 174
- WO-A1-94/28865
- WO-A1-97/46217
- JP-A- 9 110 663
- JP-A- 11 171 751
- JP-A- 11 199 455
- JP-A- 11 199 456
- JP-A- 11 246 377
- JP-A- 2001 039 842
- JP-A- 2001 302 474
- US-A- 4 327 076

## Description

### BACKGROUND OF THE INVENTION

### Field of the Invention

The present invention relates to an oral composition which has an excellent shape-holding ability and dispersibility, and does not change a taste of juice after teeth brushing and, particularly, has excellent stability with time. Moreover, the present invention relates to an oral composition having an excellent ability of a cationic antimicrobial agent to reside on a tooth surface.

### Description of the Related Art

Hitherto, a shape-holding ability and dispersibility in an oral cavity of an oral composition have been obtained by containing therein a thickening agent, which is generally and frequently used, such as carboxymethyl cellulose, methyl cellulose, hydroxyethyl cellulose, gum arabic, xanthan gum, carrageenan, sodium alginate, sodium polyacrylate and the like. In addition, an oral composition having better shape-holding ability and dispersibility in an oral cavity than those of prior art, which is prepared by containing therein finely-divided cellulose, has been proposed in JPA 58861/1993. However, such the oral composition has practical problems such as occurrence of solid-liquid separation during long term storage. In addition, in such an oral composition, sodium alkylsulfate is used as a surface active agent, which is known to change a taste of juice after teeth brushing.

On the other hand, a cationic antimicrobial agent is contained in various oral compositions in order to prevent an oral cavity disease such as a periodontal disease, dental caries and the like, because it has an excellent ability to be adsorbed to an oral tissue, an enhanced antimicrobial activity and an enhanced plaque formation-suppressing effect.

However, there was a problem in that, since the cationic antimicrobial agent has an electric charge, it forms an electrostatic complex with other anionic ingredients contained in the oral composition, and a antimicrobial activity per unit of the cationic antimicrobial agent is reduced. In response thereto, attempts have been conducted to prevent reduction of the activity per unit of the cationic antimicrobial agent, by containing a nonionic or amphoteric surface active agent or a nonionic thickening agent in the oral compositions, but sufficient effects have not been obtained yet.

JP-A-11/199455 describes a toothpaste containing crystalline cellulose with average particle diameter 100 - 200 µm as abrasive. Crystalline cellulose with average particle diameter 10 - 70 µm may also be blended. Surfactants, preferably anionic surfactants, are described as optional components. Various antimicrobial agents may also be included.

On the other hand, even the cationic antimicrobial agent exhibits a transient antimicrobial effect in many cases, and it is contemplated that an activity of the antimicrobial agent can be totally enhanced by improving an ability of the cationic antimicrobial agent to reside on a tooth surface.

### BRIEF SUMMARY OF THE INVENTION

A first object herein is to provide an oral composition which retains better shape-holding ability, is excellent in dispersibility in an oral cavity, does not change a taste of juice after teeth brushing, and does not cause solid-liquid separation during long term storage and, additionally, which has an improved ability of the cationic antimicrobial agent to reside on a tooth surface.

Moreover, a second object herein is to provide an oral composition which can effectively prevent a periodontal disease and dental caries by enhancing the ability of a cationic antimicrobial agent to reside on a tooth surface to enhance a residence antimicrobial activity of the cationic antimicrobial agent.

The present inventors studied intensively, and found that an oral composition which has an excellent shape-holding ability and dispersibility in an oral cavity, does not change a taste of juice after teeth brushing, and does not cause solid-liquid separation during long term storage, can be obtained by containing a combination of microcrystalline cellulose and a particular surface active agent.

The present inventors also found that the ability of the cationic antimicrobial agent to reside on a tooth surface is significantly enhanced by containing a specific combination of the cationic antimicrobial agent and microcrystalline cellulose.

The present invention provides:
1. An oral composition in the form of a toothpaste, wet dentifrice or liquid dentifrice comprising a cationic antimicrobial agent, and further comprising microcrystalline cellulose and one or more surface active agents selected from alkyl glucoside and fatty acid amide propyl betaine, wherein an alkyl chain of a fatty acid portion of the fatty acid amide propyl betaine is C8-C16 in length, for use in enhancing the ability of said cationic antimicrobial agent to reside on a tooth surface.
2. The oral composition according to (1), wherein the microcrystalline cellulose is contained at 0.2-10 % by weight.
3. The oral composition according to (1) or (2), wherein the surface active agent is alkyl glucoside.
4. The oral composition according to (3), wherein an alkyl chain of the alkyl glucoside is C8-C16 in length.

According to the invention, an oral composition can be provided which has an excellent shape-holding ability and dispersibility in an oral cavity, does not change a taste of juice after teeth brushing and, particularly, has excellent stability with time, and additionally has an enhanced ability of the cationic antimicrobial agent to reside on a tooth surface in addition to above characteristics.

### DETAILED DESCRIPTION OF THE INVENTION

Microcrystalline cellulose used in the invention is not particularly limited as far as it is commercially available, but microcrystalline cellulose having an average particle diameter of 10 micrometer or smaller is more preferable and microcrystalline cellulose having an average particle diameter of 2-6 micrometer is most preferable. When the average particle diameter of microcrystalline cellulose is larger than 10 micrometer, dispersibility of the oral composition in the oral cavity is deteriorated. In addition, an amount of microcrystalline cellulose to be contained is preferably 0.2-10 % by weight based on a total weight of the oral composition. When the amount of microcrystalline cellulose is smaller than 0.2 % by weight, an adequate shape-holding ability of the oral composition cannot be achieved, which is not preferable. On the other hand, when the amount of microcrystalline cellulose is larger than 10 % by weight, a viscosity of the oral composition becomes too high, which is not preferable.

The surface active agent used in the invention includes alkyl glucoside or betaine as specified, and they may be used alone or in a combination of two or more. Other surfactants are polyglycerin fatty acid ester and sucrose fatty acid ester. An amount of the surface active agent to be contained is preferably 0.5-5 % by weight based on a total weight of the oral composition. When the amount of the surface active agent to be contained is smaller than 0.5 % by weight, a foaming ability of the oral composition is reduced, and a use feeling is deteriorated, being not preferable. On the other hand, when the amount of the surface active agent to be contained is larger than 5 % by weight, a taste or a smell derived from the surface active agent becomes unnegligible, being not preferable.

Among above surface active agents, alkyl glucoside used in the present invention is not particularly limited, but an alkyl chain thereof is preferably C8-C16 in length. When the alkyl chain is shorter than C8, a bitter taste is produced in the oral composition, being not preferable. On the other hand, when the alkyl chain is longer than C16, the foaming ability of the oral composition is lowered and it becomes uncomfortable to use in some cases, being not preferable. Examples within such the chain length range include decyl glucoside, lauryl glucoside, myristyl glucoside and the like, and PLANTACARE 1200, PLANTACARE 2000 (Cognis), Oramix NS10, Oramix NS26 (SEPPIC) and the like are commercially available.

Polyglycerin fatty acid ester optionally used is not particularly limited, but an alkyl chain of a fatty acid portion thereof is preferably C8-C16 in length. When the alkyl chain is shorter than C8, a bitter taste is produced in the oral composition. On the other hand, when the alkyl chain is longer than C16, there is a tendency that the foaming ability of the oral composition is lowered. In addition, a polymerization degree of a polyglycerin portion is preferably equal to or greater than 4. When the polymerization degree is equal to or smaller than 3, there is a tendency that the foaming ability of the oral composition is lowered. Examples of such polyglycerin fatty acid ester include decaglycerin monolauric acid ester, tetraglycerin monolauric acid ester, decaglycerin monomyristic acid ester, tetraglycerin monomyristic acid ester and the like, and NIKKOL Decaglyn 1-L, NIKKOL Tetraglyn 1-L, NIKKOL Decaglyn 1-M (Nikko Chemicals, Co., Ltd.), Sunsoft Q-12W, Sunsoft Q-12T, Sunsoft Q-14W (Taiyo Kagaku Co., Ltd.) and the like are commercially available.

Sucrose fatty acid ester optionally used is not particularly limited, but an alkyl chain of a fatty acid portion thereof is preferably C8-C16 in length. When the alkyl chain is shorter than C8, a bitter taste is produced in the oral composition. On the other hand, when the alkyl chain is longer than C16, the foaming ability of the oral composition is lowered and an oily taste is produced in some cases in the oral composition. Examples of such sucrose fatty acid ester include sucrose lauric acid ester, sucrose palmitic acid ester and the like, and DK ester S series (Daiichi Kogyo Seiyaku Co., Ltd.), Ryoto sugar ester (Mitsubishi Kagaku Foods Co.) and the like are commercially available.

Betaine surface active agent used in the invention is fatty acid amide propyl betaine, which is used in view of its weak bitter taste. In addition, an alkyl chain of a fatty acid portion of fatty acid amide propyl betaine is C8-C16 in length. When the alkyl chain is shorter than C8, a bitter taste is produced in the oral composition. On the other hand, when the alkyl chain is longer than C16, the foaming ability of the oral composition is lowered and an oily taste is produced in some cases in the oral composition. Examples of fatty acid amide propyl betaine having such the chain length range include cocamidopropyl betaine, lauric acid amide propyl betaine, myristic acid amide propyl betaine and the like, and there are commercially available products such as SWANOL (Nikko Chemicals, Co., Ltd.), Obazolin (Toho Chemical Industry Co., Ltd.), RIKABION (New Japan Chemical Co., Ltd.), Tego-Betaine (Goldschmidt AG), Empigen (Albright & Wilson) and the like.

The cationic antimicrobial agent used in the invention is not particularly limited, but a quaternary ammonium salt and a biguanide antimicrobial agent are preferable, and examples thereof include, for example, the quaternary ammonium salt such as cetylpyridinium chloride, benzalkonium chloride, benzethonium chloride, distearyldimethyl ammonium chloride, stearyldimethylbenzyl ammonium chloride, stearyltrimethyl ammonium chloride, cetyltrimethyl ammonium chloride, lauryltrimethyl ammonium chloride, laurylpyridinium chloride and the like, and the biguanide antimicrobial agent such as chlorhexidine hydrochloride, chlorhexidine acetate, chlorhexidine gluconate, alexidin hydrochloride, alexidin acetate, alexidin gluconate and the like, and the like. Among them, cetylpyridinium chloride and benzalkonium chloride are more preferable, and cetylpyridinium chloride is particularly preferable. These cationic bacrtericides may be contained alone or in a combination of two or more. In addition, an mount of the cationic antimicrobial agent to be contained is preferably 0.001-10 % by weight, and more preferably 0.01-1 % by weight based on a total weight of the oral composition. When the amount of the cationic antimicrobial agent is smaller than 0.001 % by weight, a antimicrobial effect of the oral composition can not be expected. On the other hand, when the amount of the cationic antimicrobial agent is larger than 10 % by weight, an irritation to an oral mucous membrane becomes strong, being not preferable in view of safety.

The oral composition is in the form of a toothpaste, wet dentifrice or liquid dentifrice. Ingredients, for example, active ingredients, foaming agents or detergents, polishing agents, thickening agents, humectants, preservatives, flavors, sweeteners, pH adjusting agents or the like may be properly contained in the oral composition as long as they do not deteriorate the effects thereof, depending on a difference in the form of the oral composition.

Among them, examples of the active ingredient include a nonionic antimicrobial agent such as triclosan, isopropyl methylphenol and the like, a fluoride such as sodium fluoride, potassium fluoride, ammonium fluoride, stannous fluoride, sodium monofluorophosphate and the like, an enzyme such as amylase, protease, lysozyme, dextranase and the like, a vitamin such as vitamins B, C and E and the like, a potassium salt and the like.

Examples of the foaming agent or detergent include an anionic surface active agent such as sodium N-acyl sarcosinate, N-acyl glutamate, sodium N-methyl-N-acyltaurine, sodium N-methyl-N-acylalanine, sodium alpha-olefin sulfonate and the like; a nonionic surface active agent such as polyoxyethylene fatty acid ester such as polyoxyethylene sorbitan fatty acid ester such as polyoxyethylene sorbitan monolaurate, or polyoxyethylene hydrogenated castor oil, lauric acid monoethanol amide, myristic acid monoethanol amide, polyoxyethylene higher alcohol-ether, polyoxyethylene (polyoxypropylene) copolymer, polyoxyethylene (polyoxypropylene) fatty acid ester and the like; an amphoteric surface active agent such as N-alkyldiamino ethyl glycine and the like, in addition to the surface active agents as described above. But, since the oral composition of the invention contains the cationic antimicrobial agent, it is not preferable that it contains the anionic surface active agent.

Examples of the polishing agent include calcium hydrogenphosphate dihydrate or anhydrate, calcium phosphate, calcium tertiary phosphate, magnesium tertiary phosphate, calcium pyrophosphate, hydroxyapatite, insoluble sodium metaphosphate, silicic acid hydrate, silicic acid anhydrate, silica gel, precipitated silica, aluminum silicate, zirconium silicate, calcium silicate, calcium carbonate, magnesium carbonate, alumina, aluminum hydroxide, calcium sulfate, methyl polymethacrylate and the like. Among them, calcium hydrogenphosphate dihydrate and anhydrate, calcium phosphate, calcium tertiary phosphate, magnesium tertiary phosphate, calcium pyrophosphate, hydroxyapatite, calcium carbonate and magnesium carbonate are preferable.

Examples of the thickening agent include an anionic thickening agent such as sodium carboxymethyl cellulose, sodium carboxymethyl hydroxyethyl cellulose and the like, a cellulose derivative such as hydroxyethyl cellulose, hydroxypropyl cellulose and the like, natural gum such as xanthan gum, tragacanth, gum karaya, gum arabic, carrageenan and the like, a cationic thickening agent such as O-[2-hydroxy-3-(trimethylammonio)propyl] hydroxyethyl cellulose chloride and the like, in addition to microcrystalline cellulose used in the invention. Other thickening agents are gellan gum, synthetic thickening agents such as poly (vinylalcohol), sodium polyacrylate and the like, inorganic thickening agents such as viscosity-increasing silica, veegum and the like. Since the oral composition contains the cationic antimicrobial agent, it is not preferable that it contains an anionic thickening agent.

Examples of the humectant include glycerin, ethylene glycol, propylene glycol, 1,3-butylene glycol, sorbitol, polyethylene glycol, xylitol, polypropylene glycol and the like. Other humectants are maltitol, lactitol and palatinitol.

Examples of the preservative include paraoxybenzoic acid ester such as methyl paraben, propyl paraben and the like, benzoate, sodium benzoate and the like.

Examples of the flavor include menthol, carvone, eugenol, methyl salicylate, methyl eugenol, thymol, anethole, limonene, ocimene, n-decyl alcohol, citronel, alpha-terpineol, methyl acetate, citronenyl acetate, cinneole, linalool, ethyl linalool, vanillin, thyme, nutmeg, spearmint oil, peppermint oil, lemon oil, orange oil, sage oil, rosemary oil, cinnamon oil, perilla oil, wintergreen oil, cloves oil, eucalyptus oil, piment oil, tea tree oil, Davana oil and the like.

Examples of the sweetener include saccharin sodium, acesulfame potassium, stevioside, neohesperidin dihydrochalcone, glycyrrhizin, perillartine, thaumatin, aspartyl phenylalanine methyl ester, methoxycinnamic aldehyde, xylitol and the like.

Examples of the pH-adjusting agent include citric acid, phosphoric acid, malic acid, gluconic acid, maleic acid, aspartic acid, gluconic acid, succinic acid, glucuronic acid, fumaric acid, glutamic acid, adipic acid and salts thereof, hydrochloric acid, sodium hydroxide, potassium hydroxide, sodium silicate and the like.

These ingredients may be contained alone or in a combination of two or more in the oral composition.

### EXAMPLES

The invention will be further illustrated in detail by referring to the following Examples, but the present invention is not limited to the Examples. In the Examples, the term "%" means "% by weight", unless otherwise indicated.

Oral compositions were prepared according to the formulations shown in Table 1 by conventional procedures. Each composition obtained was tested for stability with time at room temperature for one month. The results thereof are shown in Table 1.

### Evaluation criteria

Stability with time after one month storage at room temperature:
○: No solid-liquid separation was observed
X: Solid-liquid separation was observed

**Table 1**

| Ingredient (%) | Comparative Example | | | | Reference Example | | | |
|---|---|---|---|---|---|---|---|---|
| | 1 | 2 | 3 | 4 | 1 | 2 | 3 | 4 |
| Microcrystalline cellulose (average particle diameter 4 micrometer | 3 | 3 | 3 | 3 | 3 | 3 | 3 | 3 |
| Hydroxyethyl cellulose | 1 | 3 | 1 | 1 | 1 | 1 | 1 | 1 |
| Xanthan gum | - | - | 1 | 1 | - | - | - | - |
| Poloxamer 238 (Pluronic F88) | 3 | 3 | 3 | - | - | - | - | - |
| Polyoxyethylene (60 E.O.) hydrogenated castor oil (HCO-60) | - | - | - | 3 | - | - | - | - |
| Lauryl glucoside | - | - | - | - | 3 | - | - | - |
| Decaglycerin lauric acid ester | - | - | - | - | - | 3 | - | - |
| Sucrose lauric acid ester | - | - | - | - | - | - | 3 | - |
| Cocamidopropyl betaine | - | - | - | - | - | - | - | 1 |
| Dicalcium phosphate | 30 | 30 | 30 | 30 | 30 | 30 | 30 | 30 |
| Flavor | 1 | 1 | 1 | 1 | 1 | 1 | 1 | 1 |
| Saccharin sodium | 0.1 | 0.1 | 0.1 | 0.1 | 0.1 | 0.1 | 0.1 | 0.1 |
| Titanium oxide | 0.3 | 0.3 | 0.3 | 0.3 | 0.3 | 0.3 | 0.3 | 0.3 |
| Concentrated glycerin | 20 | 20 | 20 | 20 | 20 | 20 | 20 | 20 |
| Purified water | remainder | remainder | remainder | remainder | remainder | remainder | remainder | remainder |
| Stability with time | X | X | X | X | ○ | ○ | ○ | ○ |

As shown in Table 1, in Comparative Examples 1-3, the oral compositions containing Pluronic F88 as the surface active agent caused solid-liquid separation even when an amount of hydroxyethyl cellulose was increased, or even when xanthan gum as another thickening agent was contained together. In addition, the oral composition caused solid-liquid separation even when HCO-60 was used as the surface active agent.

On the other hand, in Reference Examples 1-4, the oral compositions containing lauryl glucoside, polyglycerin lauric acid ester, sucrose lauric acid ester or cocamidopropyl betaine as the surface active agent, did not cause solid-liquid separation even after one month storage at room temperature, which had excellent stability with time.

### Example 5 [Reference]

An oral composition (toothpaste) of the following formulation was prepared according to the conventional procedures:

| Ingredient Name | Amount (%) |
|---|---|
| Microcrystalline cellulose | |
| (average particle diameter 3.7 micrometer) | 3.0 |
| Decyl glucoside | 2.0 |
| Silica | 30.0 |
| Sodium carboxymethyl cellulose | 2.0 |
| Tocopherol acetate | 0.05 |
| Sodium fluoride | 0.2 |

| | |
|---|---|
| Flavor | 1.0 |
| Saccharin sodium | 0.1 |
| Titanium oxide | 0.3 |
| Sorbit solution | 30.0 |
| Purified water | remainder |

The oral composition obtained had an excellent shape-holding ability and dispersibility in an oral cavity, did not change a taste of juice after teeth brushing and had excellent stability with time.

### Example 6 [Invention]

An oral composition (toothpaste) of the following formulation was prepared according to the conventional procedures:

| Ingredient Name | Amount (%) |
|---|---|
| Microcrystalline cellulose | |
| (average particle diameter 3.7 micrometer) | 3.0 |
| Cocamidopropyl betaine | 0.8 |
| Calcium hydrogenphosphate | 35.0 |
| Cetylpyridinium chloride | 0.1 |
| Hydroxyethyl cellulose | 2.0 |
| Tocopherol acetate | 0.05 |
| Sodium monofluorophosphate | 0.72 |
| Flavor | 1.0 |
| Saccharin sodium | 0.1 |

| | |
|---|---|
| Titanium oxide | 0.3 |
| Concentrated glycerin | 15.0 |
| Purified water | remainder |

The oral composition obtained had an excellent shape-holding ability and dispersibility in an oral cavity, did not change a taste of juice after teeth brushing and had excellent stability with time. In addition, the oral composition obtained had an enhanced effect of cetylpyridinium chloride to reside on a tooth surface.

### Example 7 [Reference]

An oral composition (toothpaste) of the following formulation was prepared according to the conventional procedures:

| Ingredient Name | Amount (%) |
|---|---|
| Microcrystalline cellulose | |
| (average particle diameter 3.7 micrometer) | 2.0 |
| Sucrose lauric acid ester | 2.0 |
| Calcium pyrophosphate | 35.0 |
| Xanthan gum | 0.5 |
| Sodium monofluorophosphate | 0.72 |
| Flavor | 1.0 |
| Saccharin sodium | 0.1 |
| Titanium oxide | 0.3 |
| Concentrated glycerol | 18.0 |

| | |
|---|---|
| Polyethylene glycol | 5.0 |
| Purified water | remainder |

The oral composition obtained had an excellent shape-holding ability and dispersibility in an oral cavity, did not change a taste of juice after teeth brushing and had excellent stability with time.

### Example 8 [Reference]

An oral composition (toothpaste) of the following formulation was prepared according to the conventional procedures:

| Ingredient Name | Amount (%) |
|---|---|
| Microcrystalline cellulose | |
| (average particle diameter 3.7 micrometer) | 2.0 |
| Decaglycerin lauric acid ester | 2.0 |
| Calcium carbonate | 25.0 |
| Sodium carboxymethyl cellulose | 1.0 |
| Flavor | 1.0 |
| Saccharin sodium | 0.1 |
| Titanium oxide | 0.3 |
| Concentrated glycerin | 10.0 |
| Xylitol | 10.0 |
| Purified water | remainder |

The oral composition obtained had an excellent shape-holding ability and dispersibility in an oral cavity, did not change a taste of juice after teeth brushing and had excellent stability with time.

### Example 9 [Reference]

An oral composition (gel) of the following formulation was prepared according to the conventional procedures:

| Ingredient Name | Amount (%) |
|---|---|
| Microcrystalline cellulose | |
| (average particle diameter 3.7 micrometer) | 4.0 |
| Decyl glucoside | 1.0 |
| Sodium fluoride | 0.2 |
| Concentrated glycerin | 40.0 |
| Polyethylene glycol | 5.0 |
| Propylene glycol | 8.0 |
| Flavor | 1.0 |
| Saccharin sodium | 0.1 |
| Disodium hydrogenphophate | 0.12 |
| Sodium dihydrogenphosphate | 0.01 |
| Purified water | remainder |

The oral composition obtained had an excellent shape-holding ability and dispersibility in an oral cavity, did not change a taste of juice after teeth brushing and had excellent stability with time.

### Example 10 [Reference]

An oral composition (gel) of the following formulation was prepared according to the conventional procedures:

| Ingredient Name | Amount (%) |
|---|---|
| Microcrystalline cellulose | |
| (average particle diameter 3.7 micrometer) | 5.0 |
| Myristic acid amide propyl betaine | 0.5 |
| Tetraglycerin lauric acid ester | 1.0 |
| Tocopherol acetate | 0.1 |
| Concentrated glycerin | 30.0 |
| Polyethylene glycol | 4.0 |
| 1,3-Butylene glycol | 2.0 |
| Flavor | 1.0 |
| Saccharin sodium | 0.1 |
| Disodium hydrogencitrate | 0.12 |
| Sodium dihydrogencitrate | 0.01 |
| Purified water | remainder |

The oral composition obtained had an excellent shape-holding ability and dispersibility in an oral cavity, did not change a taste of juice after teeth brushing and had excellent stability with time.

### Example 11 [Reference]

An oral composition (toothpaste) of the following formulation was prepared according to the conventional procedures:

| Ingredient Name | Amount (%) |
|---|---|
| Microcrystalline cellulose (average particle diameter 5.8 micrometer) | 0.5 |
| Lauryl glucoside | 2.5 |
| Calcium hydrogenphosphate dihydrate | 40.0 |
| Hydroxyethyl cellulose | 1.0 |
| Flavor | 1.0 |
| Saccharin sodium | 0.2 |
| Sorbitol | 25.0 |
| Purified water | remainder |

The oral composition obtained had an excellent shape-holding ability and dispersibility in an oral cavity, did not change a taste of juice after teeth brushing and had excellent stability with time.

### Example 12 [Reference]

An oral composition (toothpaste) of the following formulation was prepared according to the conventional procedures:

| Ingredient Name | Amount (%) |
|---|---|
| Microcrystalline cellulose (average particle diameter 5.8 micrometer) | 2.0 |
| Decyl glucoside | 1.5 |

| | |
|---|---|
| Silicic acid hydrate | 20.0 |
| Carrageenan | 1.0 |
| Flavor | 1.0 |
| Saccharin sodium | 0.1 |
| Sorbitol | 15.0 |
| Concentrated glycerin | 10.0 |
| Purified water | remainder |

The oral composition obtained had an excellent shape-holding ability and dispersibility in an oral cavity, did not change a taste of juice after teeth brushing and had excellent stability with time.

### Example 13 [Reference]

An oral composition (toothpaste) of the following formulation was prepared according to the conventional procedures:

| Ingredient Name | Amount (%) |
|---|---|
| Microcrystalline cellulose (average particle diameter 8.6 micrometer) | 1.0 |
| Cocamidopropyl betaine | 0.8 |
| Silica | 15.0 |
| Aluminum hydroxide | 5.0 |
| Sodium polyacrylate | 0.5 |
| Flavor | 1.0 |
| Saccharin sodium | 0.2 |

| | |
|---|---|
| Polyethylene glycol | 5.0 |
| Concentrated glycerin | 10.0 |
| Purified water | remainder |

The oral composition obtained had an excellent shape-holding ability and dispersibility in an oral cavity, did not change a taste of juice after teeth brushing and had excellent stability with time.

### EXPERIMENTS

### Method of Experiment

### Measurement of the amount of cetylpyridinium chloride residing on hydroxyapatite powder

50 mg of hydroxyapatite (DNA Grade Bio-Gel HTP; manufactured by BIO-RAD) was immersed in 2 ml of human saliva, which had been sterilized with ultraviolet rays, at 37 centigrade for 15 hours to allow to form an artificial pellicle on a hydroxyapatite surface. Thereafter, a mixture of hydroxyapatite and human saliva was centrifuged (3000 rpm, 10 min) and a supernatant was discarded. Then, residual hydroxyapatite was immersed at 37 centigrade for 15 minutes in 2 ml of a supernatant of a four times-diluted slurry from each of the oral compositions of Examples 14-18 (Examples 14, 15, 17 and 18 embody the claimed invention) and Comparative Examples 5-8, in which 0.3 % by weight of cetylpyridinium chloride (CPC) and various amounts and various kinds of thickening agents and surface active agents had been contained. Then, a mixture was centrifuged (3000 rpm, 10 minutes), and a supernatant was discarded. Then, 2 ml of fresh distilled water was added to the residue, the mixture was stirred and centrifuged (3000 rpm, 10 min), and the supernatant was discarded. Again, 2 ml of fresh distilled water was added to the residue, the mixture was stirred and centrifuged (3000 rpm, 10 minutes), and the supernatant was discarded. Next, cetylpyridinium chloride which had adsorbed onto hydroxyapatite was extracted with an extraction solution as described below, and an amount of cetylpyridinium chloride residing on 50 mg of hydroxyapatite was quantitatively measured with high performance liquid chromatography. The results thereof are shown in Table 2.

The extraction solution was prepared by mixing a solution in which 2.88 g of sodium lauryl sulfate had been dissolved per 1 liter of 0.02 M citrate buffer, pH 3 with acetonitrile in a ratio of 1:3.

**Table 2**

| Ingredient | Example 14 | Example 15 | Example 16 (Reference) | Example 17 | Example 18 | Comparative Ex. 5 | Comparative Ex. 6 | Comparative Ex. 7 | Comparative Ex. 8 |
|---|---|---|---|---|---|---|---|---|---|
| Cetylpyridinium chloride | 0.3 | 0.3 | 0.3 | 0.3 | 0.3 | 0.3 | 0.3 | 0.3 | 0.3 |
| Microcrystalline cellulose | 4 | 4 | 4 | 3 | 3 | - | - | - | - |
| Hydroxyethyl cellulose | - | - | - | 1 | 1 | 4 | 4 | 4 | - |
| Sodium carboxymethyl cellulose | - | - | - | - | - | - | - | - | 1.5 |
| Decyl glucoside | 1 | - | - | 1 | - | 1 | - | - | - |
| Lauryl glucoside | - | - | - | - | 2 | - | - | - | - |
| Cocamidopropyl betaine | - | 1 | - | - | - | - | 1 | - | 1 |
| Polyoxyethylene castor oil | - | - | 1 | - | - | - | - | 1 | - |
| Dicalcium phosphate dihydrate | 35 | 35 | 35 | 35 | 35 | 35 | 35 | 35 | 35 |
| Saccharin sodium | 0.3 | 0.3 | 0.3 | 0.3 | 0.3 | 0.3 | 0.3 | 0.3 | 0.3 |
| Titanium oxide | 0.3 | 0.3 | 0.3 | 0.3 | 0.3 | 0.3 | 0.3 | 0.3 | 0.3 |
| Flavor | 0.9 | 0.9 | 0.9 | 0.9 | 0.9 | 0.9 | 0.9 | 0.9 | 0.9 |
| Glycerin | 15 | 15 | 15 | 15 | 15 | 15 | 15 | 15 | 15 |
| Ion exchanged water | remainder | remainder | remainder | remainder | remainder | remainder | remainder | remainder | remainder |
| CPC remaining amount (microgram/50 mg hydroxyapatite) | 863 | 583 | 194 | 904 | 517 | 409 | 367 | 180 | 48 |

From the results in Table 2, it is found that an amount of cetylpyridinium chloride residing on a tooth surface is significantly increased with the oral compositions of Examples 14-18 (Examples 14, 15, 17 and 18 embody the claimed invention), in which cetylpyridinium chloride and microcrystalline cellulose have been specifically combined, as compared with those of Comparative Examples 5-8, in which the same amount of cetylpyridinium chloride and other cellulose derivatives have been combined. Moreover, it is also found that alkyl glucoside and betaine are preferable as the surface active agent to be contained in addition to the above ingredients, because they increase an amount of cetylpyridinium chloride residing on a tooth surface, as compared with other surface active agents.

### Example 19 [Invention]

An oral composition (toothpaste) of the following formulation was prepared according to the conventional procedures:

| Ingredient Name | Amount (%) |
|---|---|
| Benzethonium chloride | 0.1 |
| Microcrystalline cellulose | |
| (average particle diameter 3.7 micrometer) | 2.0 |
| Triclosan | 0.1 |
| Hydroxypropylmethyl cellulose | 1.0 |
| Lauryl glucoside | 2.0 |
| Calcium carbonate | 40.0 |

| | |
|---|---|
| Titanium oxide | 0.2 |
| Saccharin sodium | 0.2 |
| Sorbit solution | 30.0 |
| Flavor | 1.0 |
| Purified water | remainder |

The oral composition obtained could increase an amount of the cationic antimicrobial agent residing on a tooth surface and could effectively prevent an oral cavity disease such as a periodontal disease, dental caries and the like.

### Example 20 [Invention]

An oral composition (toothpaste) of the following formulation was prepared according to the conventional procedures:

| Ingredient Name | Amount (%) |
|---|---|
| Cetylpyridinium chloride | 0.1 |
| Microcrystalline cellulose | |
| (average particle diameter 3.7 micrometer) | 3.0 |
| Potassium nitrate | 1.0 |
| Hydroxyethyl cellulose | 2.0 |
| Cocamidopropyl betaine | 1.0 |
| Concentrated glycerin | 10.0 |
| Sorbit solution | 10.0 |
| Titanium oxide | 0.3 |

| | |
|---|---|
| Stevioside | 0.2 |
| Sodium benzoate | 0.1 |
| Xylitol | 10.0 |
| Flavor | 0.8 |
| Purified water | remainder |

The oral composition obtained could increase an amount of the cationic antimicrobial agent residing on a tooth surface and could effectively prevent an oral cavity disease such as a periodontal disease, dental caries and the like.

### Example 21 [Reference]

An oral composition (gel) of the following formulation was prepared according to the conventional procedures:

| Ingredient Name | Amount (%) |
|---|---|
| Cetylpyridinium chloride | 0.1 |
| Microcrystalline cellulose | |
| (average particle diameter 3.7 micrometer) | 4.0 |
| Decyl glucoside | 1.0 |
| Concentrated glycerin | 40.0 |
| Polyethylene glycol | 5.0 |
| Propylene glycol | 3.0 |
| Flavor | 1.0 |
| Saccharin sodium | 0.1 |

| | |
|---|---|
| Disodium hydrogenphophate | 0.12 |
| Sodium dihydrogenphosphate | 0.01 |
| Purified water | remainder |

The oral composition obtained could increase an amount of the cationic antimicrobial agent residing on a tooth surface and could effectively prevent an oral cavity disease such as a periodontal disease, dental caries and the like.

### Example 22 [Reference]

An oral composition (gel) of the following formulation was prepared according to the conventional procedures:

| Ingredient Name | Amount (%) |
|---|---|
| Chlorhexidine hydrochloride | 0.2 |
| Microcrystalline cellulose | |
| (average particle diameter 3.7 micrometer) | 5.0 |
| Myristic acid amide propyl betaine | 0.5 |
| Tetraglycerin lauric acid ester | 1.0 |
| Tocopherol acetate | 0.1 |
| Concentrated glycerin | 30.0 |
| Polyethylene glycol | 4.0 |
| 1,3-Butylene glycol | 2.0 |
| Flavor | 1.0 |
| Saccharin sodium | 0.1 |

| | |
|---|---|
| Disodium hydrogencitrate | 0.12 |
| Sodium dihydrogencitrate | 0.01 |
| Purified water | remainder |

The oral composition obtained could increase an amount of the cationic antimicrobial agent residing on a tooth surface and could effectively prevent an oral cavity disease such as a periodontal disease, dental caries and the like.

### Example 23 [Reference]

An oral composition (toothpaste) of the following formulation was prepared according to the conventional procedures:

| Ingredient Name | Amount (%) |
|---|---|
| Benzalkonium chloride | 0.05 |
| Microcrystalline cellulose | |
| (average particle diameter 5.8 micrometer) | 0.5 |
| Sucrose myristic acid ester | 4.0 |
| Magnesium carbonate | 5.0 |
| Calcium carbonate | 12.0 |
| Guar gum | 1.0 |
| Flavor | 1.0 |
| Saccharin sodium | 0.2 |
| Concentrated glycerin | 20.0 |
| Purified water | remainder |

The oral composition obtained could increase an amount of the cationic antimicrobial agent residing on a tooth surface and could effectively prevent an oral cavity disease such as a periodontal disease, dental caries and the like.

### Example 24 [Reference]

An oral composition (gel) of the following formulation was prepared according to the conventional procedures:

| Ingredient Name | Amount (%) |
|---|---|
| Chlorhexidine gluconate | 0.2 |
| Microcrystalline cellulose (average particle diameter 8.6 micrometer) | 5.0 |
| Myristyl glucoside | 4.0 |
| Hydroxypropylmethyl cellulose | 1.0 |
| Flavor | 0.5 |
| Saccharin sodium | 0.2 |
| Concentrated glycerin | 20.0 |
| Propylene glycol | 3.0 |
| Purified water | remainder |

The oral composition obtained could increase an amount of the cationic antimicrobial agent residing on a tooth surface and could effectively prevent an oral cavity disease such as a periodontal disease, dental caries and the like.

According to the invention, an oral composition can be provided which has an excellent shape-holding ability and dispersibility in an oral cavity, does not change a taste of juice after teeth brushing, and particularly, excellent stability with time, or which has an enhanced ability of a cationic antimicrobial agent to reside on a tooth surface.

Moreover, an oral composition can be provided which can significantly increase an amount of a cationic antimicrobial agent residing on a tooth surface and effectively prevent an oral cavity disease such as a periodontal disease, dental caries and the like.

## Claims

1. An oral composition in the form of a toothpaste, wet dentifrice or liquid dentifrice comprising a cationic antimicrobial agent, and further comprising microcrystalline cellulose and one or more surface active agents selected from alkyl glucoside and fatty acid amide propyl betaine, wherein an alkyl chain of a fatty acid portion of the fatty acid amide propyl betaine is C8-C16 in length, for use in enhancing the ability of said cationic antimicrobial agent to reside on a tooth surface.

2. An oral composition according to claim 1 wherein the microcrystalline cellulose is contained at 0.2 to 10 % by weight.

3. An oral composition according to claim 1 or 2 wherein the microcrystalline cellulose has an average particle size of 10 µm or smaller.

4. An oral composition according to claim 1, 2 or 3 wherein the surface active agent is alkyl glucoside.

5. An oral composition according to claim 4 wherein an alkyl chain of the alkyl glucoside is C8-C16 in length.

6. An oral composition according to any one of claims 1 to 5 in which the amount of said surface active agent is 0.5 to 5% by weight based on the total weight of the oral composition.

7. An oral composition according to any one of claims 1 to 6 wherein the cationic antimicrobial agent is a quaternary ammonium salt or biguanide antimicrobial agent.

8. An oral composition according to claim 7 wherein the cationic antimicrobial agent is cetylpyridinium chloride or benzalkonium chloride.

## Patentansprüche

1. Eine orale Zusammensetzung in Form einer Zahncreme, nassem Zahnpflegemittel oder flüssigem Zahnpflegemittel, umfassend ein kationisches antimikrobielles Mittel, und ferner umfassend mikrokristalline Cellulose und ein oder mehrere grenzflächenaktive Mittel, ausgewählt aus Alkylglucosid und Fettsäureamid-Propylbetain, wobei eine Alkylkette eines Fettsäureanteils des Fettsäureamid-Propylbetains eine Länge von C8-C16 aufweist, zur Verwendung bei der Verbesserung der Fähigkeit des kationischen antimikrobiellen Mittels, sich auf einer Zahnoberfläche zu befinden.

2. Eine orale Zusammensetzung gemäß Anspruch 1, wobei die mikrokristalline Cellulose mit 0,2 bis 10 Gew.-% enthalten ist.

3. Eine orale Zusammensetzung gemäß Anspruch 1 oder 2, wobei die mikrokristalline Cellulose eine durchschnittliche Teilchengröße von 10 µm oder kleiner aufweist.

4. Eine orale Zusammensetzung gemäß Anspruch 1, 2 oder 3, wobei das grenzflächenaktive Mittel Alkylglucosid ist.

5. Eine orale Zusammensetzung gemäß Anspruch 4, wobei eine Alkylkette des Alkylglucosids eine Länge von C8-C16 aufweist.

6. Eine orale Zusammensetzung gemäß einem der Ansprüche 1 bis 5, in welcher die Menge des grenzflächenaktiven Mittels 0,5 bis 5 Gew.-%, bezogen auf das Gesamtgewicht der oralen Zusammensetzung, beträgt.

7. Eine orale Zusammensetzung gemäß einem der Ansprüche 1 bis 6, wobei das kationische antimikrobielle Mittel ein quaternäres Ammoniumsalz oder Biguanid als antimikrobielles Mittel ist.

8. Eine orale Zusammensetzung gemäß Anspruch 7, wobei das kationische antimikrobielle Mittel Cetylpyridiniumchlorid oder Benzalkoniumchlorid ist.

## Revendications

1. Composition orale sous la forme d'une pâte dentifrice, d'un dentifrice humide ou d'un dentifrice liquide comprenant un agent antimicrobien cationique et comprenant en outre de la cellulose microcristalline et un ou plusieurs agents tensioactifs choisis parmi un alkylglucoside et une propylbétaïne d'amide d'acide gras, une chaîne alkyle d'une partie acide gras de la propylbétaïne d'amide d'acide gras ayant une longueur de C8 à C16, pour une utilisation dans l'amélioration de la capacité dudit agent antimicrobien cationique à se fixer sur une surface d'une dent.

2. Composition orale selon la revendication 1, dans laquelle la cellulose microcristalline est contenue à hauteur de 0,2 à 10 % en poids.

3. Composition orale selon la revendication 1 ou 2, dans laquelle la cellulose microcristalline a une taille de particule moyenne inférieure ou égale à 10 µm.

4. Composition orale selon la revendication 1, 2 ou 3, dans laquelle l'agent tensioactif est un alkylglucoside.

5. Composition orale selon la revendication 4, dans laquelle une chaine alkyle de l'alkylglucoside a une longueur en C8 à C16.

6. Composition orale selon l'une quelconque des revendications 1 à 5, dans laquelle la quantité dudit agent tensioactif est de 0,5 à 5 % en poids par rapport au poids total de la composition orale.

7. Composition orale selon l'une quelconque des revendications 1 à 6, dans laquelle l'agent antimicrobien cationique est un sel d'ammonium quaternaire ou un agent antimicrobien de biguanide.

8. Composition orale selon la revendication 7, dans laquelle l'agent antimicrobien cationique est le chlorure de cétylpyridinium ou le chlorure de benzalkonium.
